# EUROPEAN PATENT SPECIFICATION

(11) **EP 3 873 511 B1**
(45) Date of publication and mention of the grant of the patent: **07.12.2022**
(21) Application number: 19801491.2
(22) Date of filing: 28.10.2019
(51) Int. Cl.: A61K 38/44, A61P 25/28, A61P 25/14, C12N 15/861, C12N 15/864

(54) **EXPRESSION VECTOR FOR CHOLESTEROL 24-HYDROLASE IN THERAPY OF AMYOTROPHIC LATERAL SCLEROSIS**
EXPRESSIONSVEKTOR FÜR CHOLESTERIN-24-HYDROLASE IN DER THERAPIE VON AMYOTROPHER LATERALSKLEROSE
VECTEUR D'EXPRESSION POUR LE CHOLESTÉROL 24-HYDROLASE DANS LA THÉRAPIE DE LA SCLÉROSE LATÉRALE AMYOTROPHIQUE

(30) Priority: 29.10.2018 EP 18306411
(43) Date of publication of application: 08.09.2021
(73) Proprietor: Institut National de la Santé et de la Recherche Médicale (INSERM), 75013 Paris (FR)
(72) Inventor: PIGUET, Françoise, 92290 Chatenay Malabry (FR); CARTIER-LACAVE, Nathalie, 75012 PARIS (FR)
(74) Representative: Cabinet Becker et Associés
(86) International application number: PCT/EP2019/079365
(87) International publication number: WO 2020/089154

(56) References cited:
- WO-A1-2009/034127
- WO-A1-2012/049314
- WO-A1-2018/138371
- MASAHISA KATSUNO ET AL: "Getting a handle on Huntington's disease: the case for cholesterol", NATURE MEDICINE, vol. 15, no. 3, 1 March 2009 (2009-03-01), pages 253-254, XP055015270, ISSN: 1078-8956, DOI: 10.1038/nm0309-253
- LYDIE BOUSSICAULT ET AL: "CYP46A1, the rate-limiting enzyme for cholesterol degradation, is neuroprotective in Huntington's disease", BRAIN., vol. 139, no. 3, 29 January 2016 (2016-01-29), pages 953-970, XP055459042, GB ISSN: 0006-8950, DOI: 10.1093/brain/awv384

## Description

The present invention relates to treatment of amyotrophic lateral sclerosis.

### Background of the invention

Amyotrophic lateral sclerosis (ALS) is a rare neurological disease that belongs to the group of motor neurons disorders that mainly involve the neurons responsible of controlling voluntary muscle movement, like chewing, walking and talking (Zarei S et al. 2015). ALS is characterized by gradual deterioration and death of motoneurons, that extend from the brain to the spinal cord and to the muscles throughout the body. ALS involves both the upper motor neurons (messages from motor neurons to the brain transmitted to the spinal cord) and lower motor neurons (motor nuclei in the brain) that degenerate and consequently stop to send messages to muscles that gradually weaken, start to twitch and gets atrophied (Rowland LP et al. 2001).

Most people suffering of ALS die within 3 to 5 years from the first symptom appearance, usually from respiratory failure. Only 10% of people with ALS survive more than 10 years after symptom onset.

In ALS several potential risk factors have been associated: age, usually symptoms appear between 55 and 75 years, gender, ALS is slightly more prevalent in men and race and ethnicity, ALS most likely developed by Caucasian and non-hispanics.

However, the vast majority of ALS cases (90%) are sporadic. In contrast around 10% of ALS cases are familial (FALS). For these familial cases several genes have been implicated: C9ORF72, SOD1, FUS, TARDBP and more recently SMCR8 (Greenway et al. 2006; Kabashi et al. 2008; Kaur et al. 2016; Turner MR et al. 2017; Ticozzi et al. 2011; Valdmanis PN et al. 2008).

Concerning ALS pathophysiology, several aspects are under consideration, one of the principal theories is related with RNA processing, in particular based on the finding of TDP-43 inclusion in most ALS cases as well as genes such as TARDBP and FUS as genetic causes of ALS. Both of these genes are implicated in pre-mRNA splicing and RNA transport and translation. Pre-mRNA that content C9orf72 repeats could sequester nuclear RNA binding proteins and thus make them unavailable for correct splicing of other mRNAs. Another well recognized aspect of ALS is protein aggregation including SOD1, TDP43, and FUS which can be observed both in ALS patients and animal models. The aggregates are proposed to disturb normal protein homeostasis and induce cellular stress. Moreover, protein aggregates could sequester RNA or other proteins essential for normal cellular functions.

Up to now, there is no known cure for ALS. There are only two drugs approved by FDA for ALS: riluzole and edaravone. Riluzole is believed to reduce damage to motor neurons acting through decreasing levels of glutamate. Riluzole prolongs survival for few months but do not reverse the damages already present in neurons (Zoccolella et al. 2007). Concerning Edaravone, it has only been shown to decline the clinical assessment of daily impairment (Brooks et al. 2018). There is thus an acute need to develop new strategies for therapy in ALS.

### Summary of the invention

The inventors now propose to counteract ALS by modulating the cholesterol metabolism pathway, more specifically by means of a vector comprising cholesterol 24-hydroxylase encoding nucleic acid that expresses cholesterol 24-hydroxylase in the target cells.

It is therefore an object of the present invention to provide a vector for use in the treatment of amyotrophic lateral sclerosis, which vector comprises cholesterol 24-hydroxylase encoding nucleic acid.

In an embodiment, the vector comprises a nucleic acid sequence that encodes the amino acid sequence SEQ ID N°2. Alternatively, the vector comprises the nucleic acid sequence SEQ ID N°1.

In an embodiment, the vector is selected from the group of adenovirus, lentivirus, retrovirus, herpes-virus and Adeno-Associated Virus (AAV) vectors, preferably an AAV vector, more preferably an AAV9, AAV10 (AAVrh.10) or AAVPHP.eB vector, even more preferably an AAVPHP.eB.

In an embodiment, the vector is administered directly into the brain and/or spinal cord of the patient, preferably to spinal cord and/or motor cortex.

It is another object of the invention to provide a pharmaceutical composition for use in the treatment of amyotrophic lateral sclerosis, which comprises a vector comprising cholesterol 24-hydroxylase encoding nucleic acid.

### Brief description of the Figures

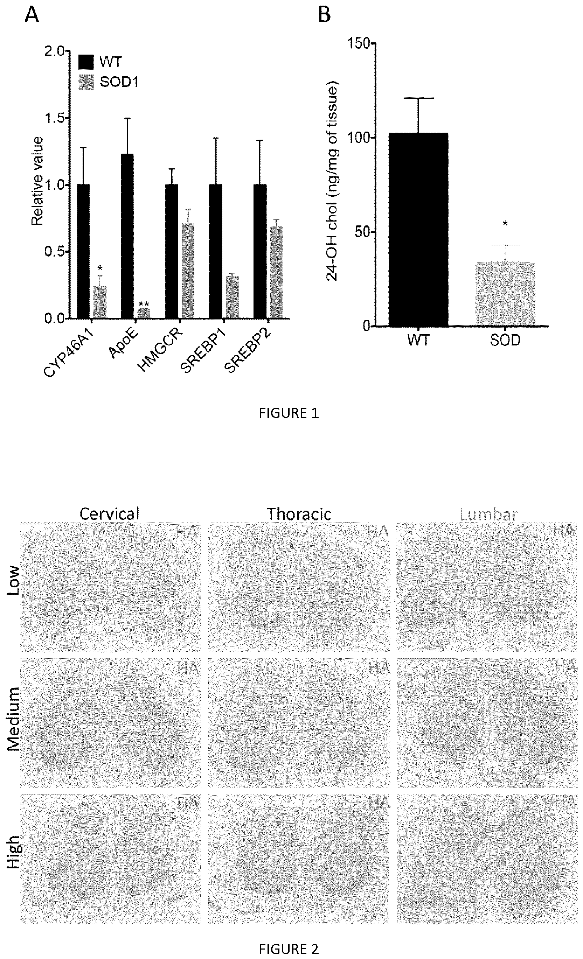

Figure 1- **mRNA levels of genes involved in cholesterol metabolism at 8 weeks in lumbar spinal cord and levels of 24-OH cholesterol at 15 weeks in spinal cord of WT and SOD1^{G93A} animals.** (A) mRNA was extracted from the lumbar spinal cord of 8 week-old WT and SOD1^{G93A} mice. mRNA levels were normalized to actin housekeeping gene. Data are represented as mean ± SEM (n = 4-5 per group). (B) Lumbar spinal cord 24S-hydroxycholesterol content in SOD1^{G93A} mice assessed by UPLC-HRMS. Data are represented as mean ± SEM (n = 7-9 per group). Statistical analysis: student t-test. ^{∗}p<0.05, ^{∗∗}p<0.01.
Figure 2- **Evaluation of CYP46A1-HA expression after intravenous delivery of AAVPHP.eB-CYP46A1-HA in WT animals at 8 weeks and analysis 3 weeks after injection of low, medium or high dose.** HA staining on cervical, thoracic and lumbar section of the spinal cord.

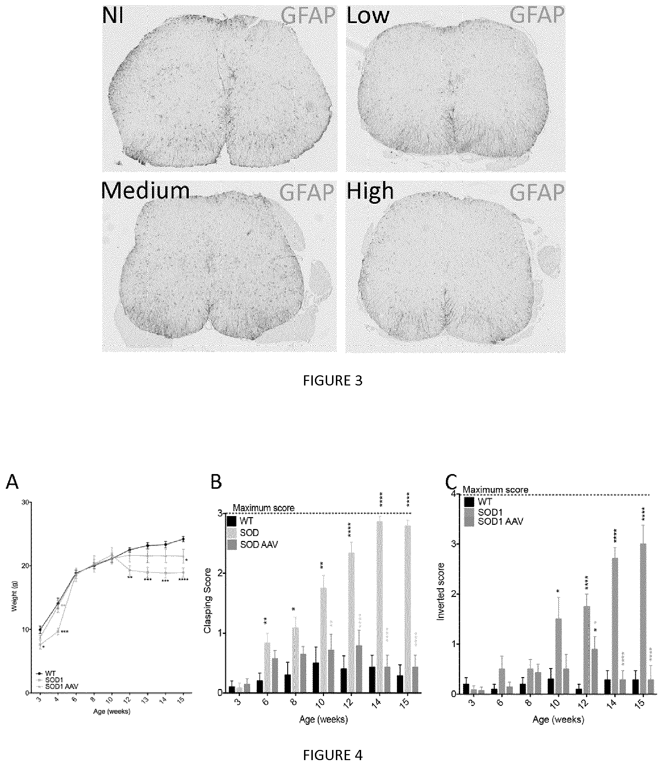

Figure 3 - **Evaluation of inflammation after intravenous delivery of AAVPHP.eB-CYP46A1-HA in WT animals at 8 weeks and analysis 3 weeks after injection of low, medium or high dose.** GFAP staining on lumbar section of the spinal cord. NI: Non injected
Figure 4 - **Behavioral evaluation of SOD1 mice after intravenous administration of AAVPHP.eB- CYP46A1 at preventive stage (3 weeks).** (A) Weight follow up, (B) Clasping test and (C). Inverted test. Results are presented as mean ± SEM. ^{∗}p<0.05, ^{∗∗}p<0.01, ^{∗∗∗}p<0.001, ^{∗∗∗∗}p<0.0001. Black ^{∗} corresponds to p value vs WT animals and grey to SOD1 animals.

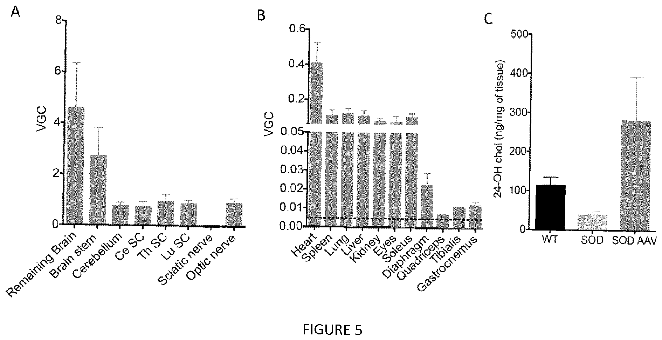

Figure 5- **Biodistribution of AAVPHP.eB-CYP46A1 after preventive treatment in SOD1 animals and evaluation of target engagement through 24OH cholesterol levels quantification at 15 weeks of age after preventive treatment.** (A) Biodistribution in central nervous system and (B) peripheral organs and (C) Lumbar spinal cord 24S-hydroxycholesterol content in SOD1G93A mice assessed by UPLC-HRMS. Results are presented as mean ± SEM.

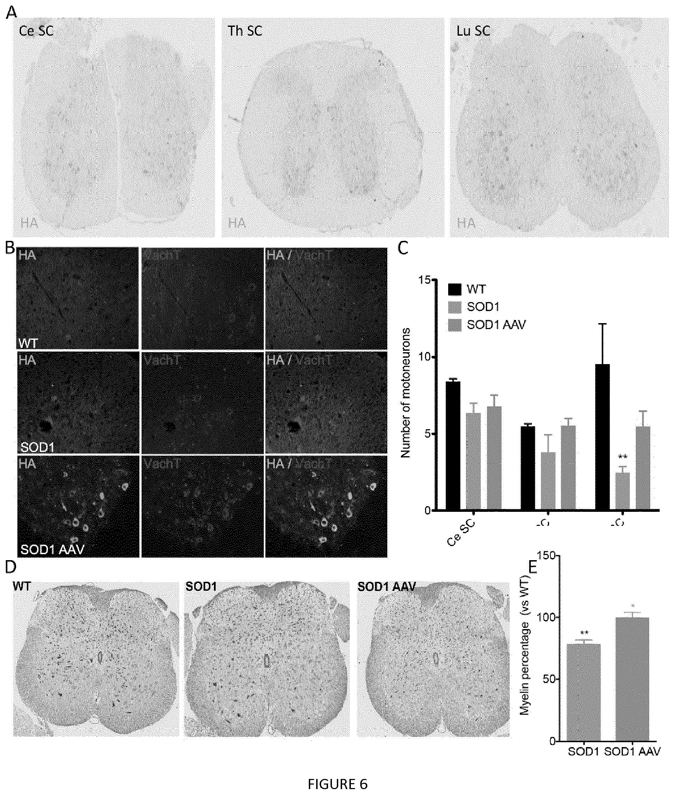

Figure 6 **- Evaluation at 15 weeks of age of CYP46A1-HA expression and histological analysis of the spinal cord after intravenous delivery of AAVPHP.eB-CYP46A1** as a **preventive treatment.** (A) HA staining on cervical, thoracic and lumbar section of the spinal cord of SOD1 animals treated with AAV; (B) Co staining for HA and VachT on cervical, thoracic and lumbar section of the spinal cord of WT, SOD1 and SOD1 AAV animals; (C) Quantification of motoneurons number; (D) Luxol staining on Lumbar section of the spinal cord and (E) evaluation of myelin percentage. Results are presented as mean ± SEM. ^{∗}p<0.05, ^{∗∗}p<0.01. Black ^{∗} corresponds to p value vs WT animals and grey to SOD1 animals.

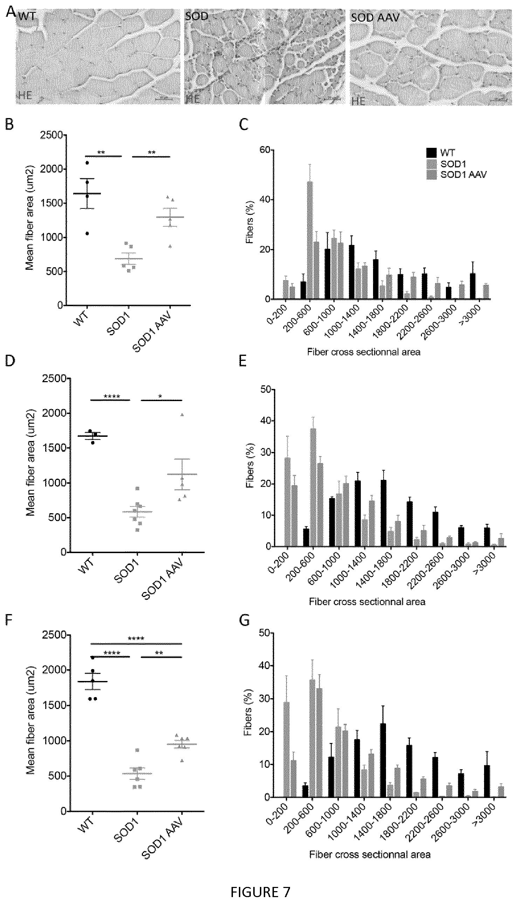

Figure 7 - **Improvement of muscular phenotype in AAV treated SOD1 animals after preventive treatment.** (A) Hematein Eosin coloration to analyze fiber size in muscle. Mean fibers areas are presented in tibialis (B), gastrocnemius (D) and quadriceps (F) as well as repartition of fiber percentage according to their cross section size in tibialis (C), gastrocnemius (E) and quadriceps (G). Results are presented as mean ± SEM. ^{∗}p<0.05, ^{∗∗}p<0.01.

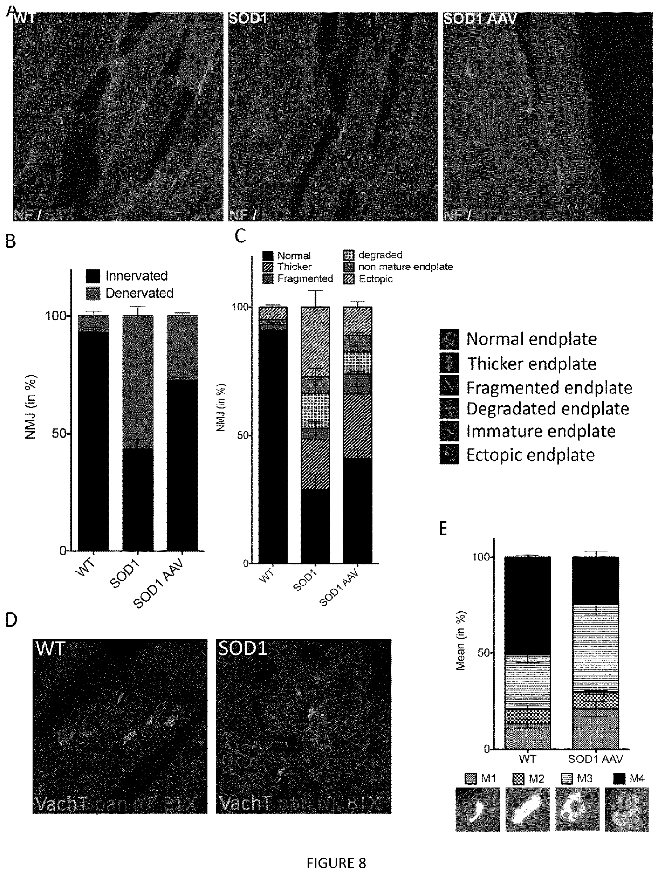

Figure 8 - **Preservation of neuromuscular junctions in AAV treated SOD1 animals after preventive treatment.** (A) Co staining of neurofilament (NF) and bungarotoxin (BTX) in 15 weeks-old WT, SOD1 and SOD1 treated animals after intravenous delivery of AAVPHP.eB-CYP46A1. (B-C) Scoring of NMJ according to their state of innervation and their integrity at 15 weeks of age. (D) evaluation of NMJ formation at 3 weeks in WT and SOD1 animals using triple staining for pan NF, bungarotoxine and VachT. (E) quantification of NMJ based on their state of maturation M1 to M4 at 3 weeks of age.

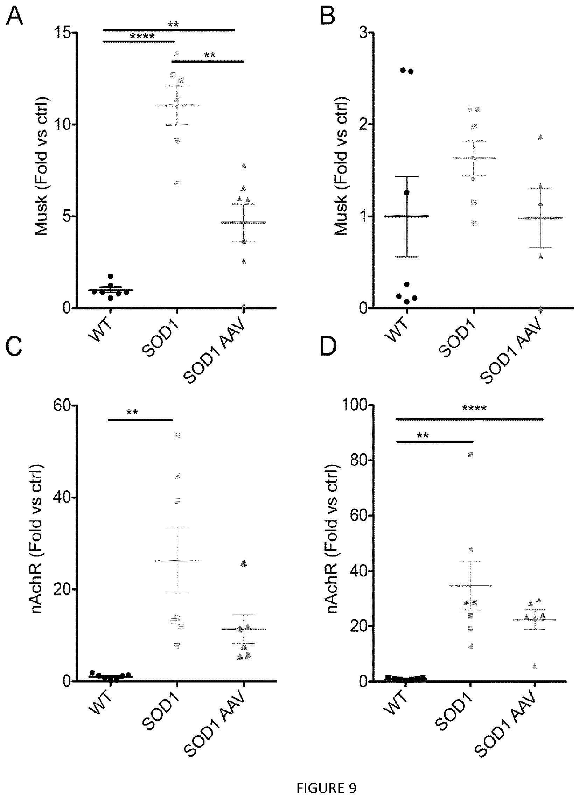

Figure 9 - **Molecular analysis of NMJ at 15 weeks in preventive treatment.** Analysis of Musk expression in tibialis (A) and gastrocnemius (B) and nAchR expression quantification in tibialis (C) and gastrocnemius (D).

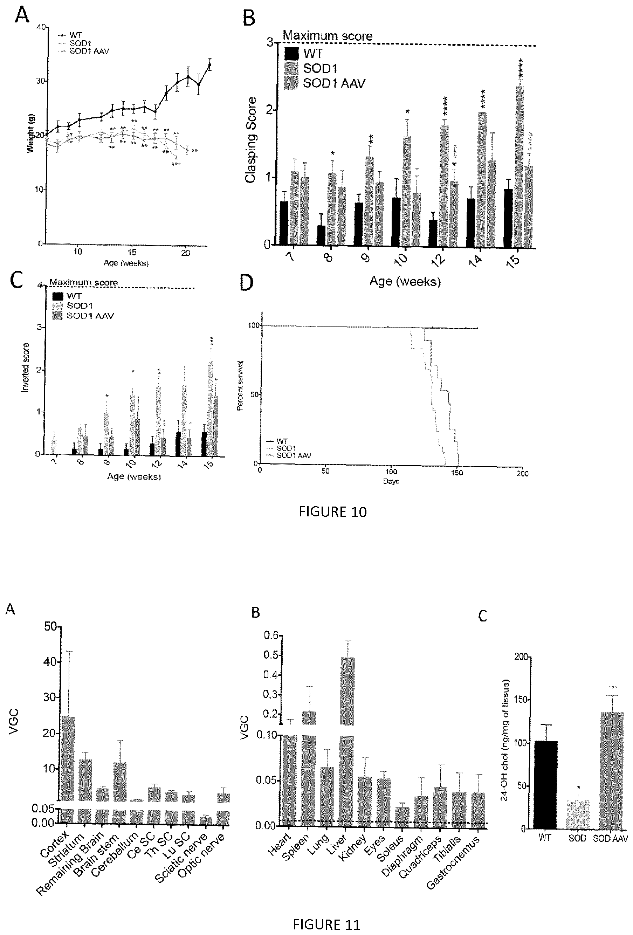

Figure 10 - **Behavioral evaluation of SOD1 mice after intravenous administration of AAVPHP.eB- CYP46A1 at curative stage (8 weeks).** (A) Weight follow up, (B) Clasping test, (C) Inverted test and (D) survival. Results are presented as mean ± SEM. ^{∗}p<0.05, ^{∗∗}p<0.01, ^{∗∗∗}p<0.001, ^{∗∗∗∗}p<0.0001. Black ^{∗} corresponds to p value vs WT animals and grey to SOD1 animals.
Figure 11 - **Biodistribution of AAVPHP.eB-CYP46A1 after preventive treatment in SOD1 animals and evaluation of target engagement through 24OH cholesterol levels quantification at 15 weeks of age after curative treatment.** Biodistribution in (A) central nervous system and (B) peripheral organs and (C) Lumbar spinal cord of 24S-hydroxycholesterol content in SOD1G93A mice assessed by UPLC-HRMS. Results are presented as mean ± SEM.

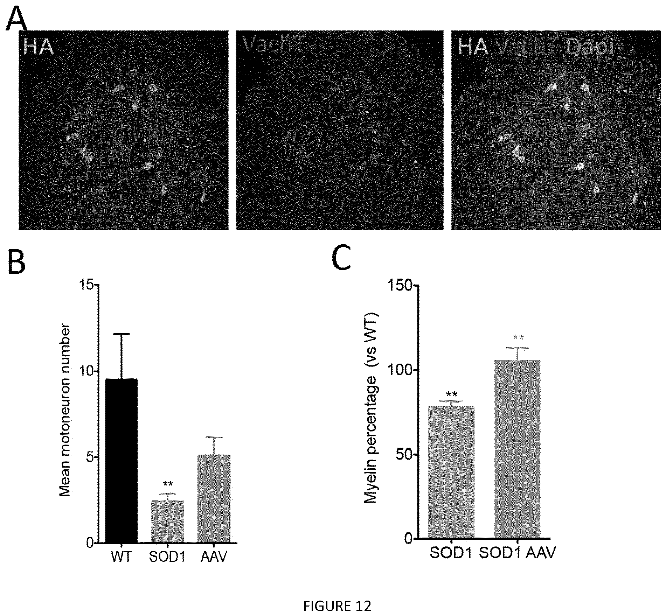

Figure 12 - **Evaluation at 15 weeks of age of CYP46A1-HA expression and histological analysis of the spinal cord after intravenous delivery of AAVPHP.eB-CYP46A1 as a curative treatment.** (A) Co staining for HA and VachT on lumbar section of the spinal cord of WT, SOD1 and SOD1 AAV animals. (B) Quantification of motoneurons number on lumbar spinal cord section and (C) Luxol staining on Lumbar section of the spinal cord. Results are presented as mean ± SEM. ^{∗}p<0.05, ^{∗∗}p<0.01. Black ^{∗} corresponds to p value vs WT animals and grey to SOD1 animals.

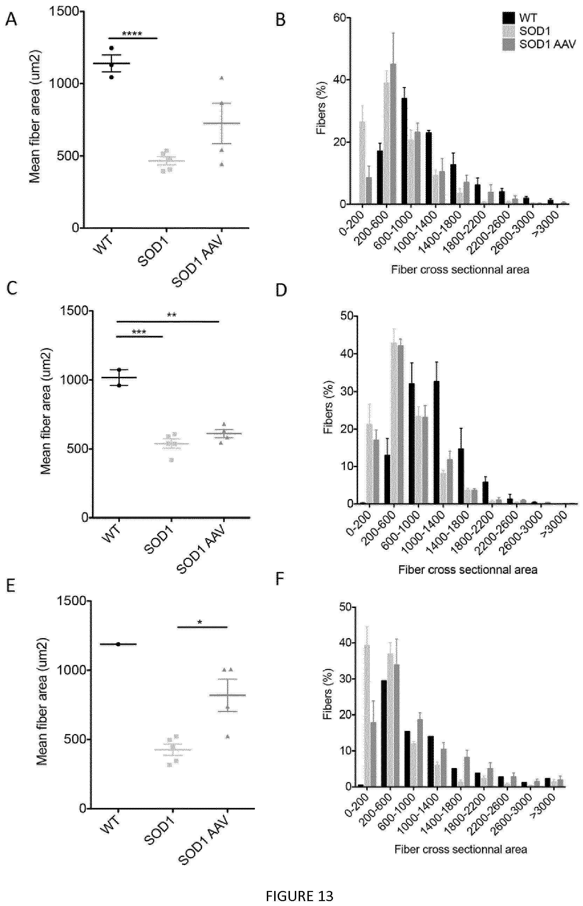

Figure 13 - **Partial correction of muscular phenotype in AAV treated SOD1 animals after curative treatment.** Mean fibers areas are presented in tibialis (A), gastrocnemius (C) and quadriceps (E) as well as repartition of fibers percentage according to their cross-section size in tibialis (B), gastrocnemius (D) and quadriceps (F). Results are presented as mean ± SEM. ^{∗}p<0.05, ^{∗∗}p<0.01.

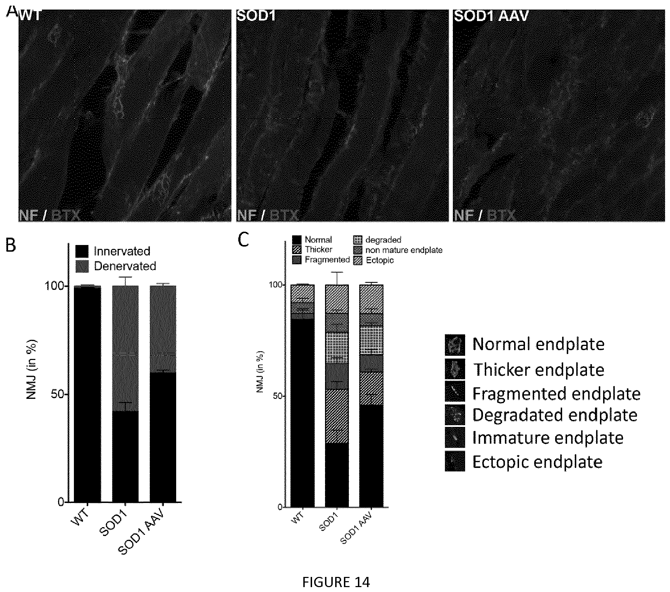

Figure 14 - **Preservation of neuromuscular junctions in AAV treated SOD1 animals after curative treatment.** (A) Co staining of neurofilament (NF) and bungarotoxin (BTX) in 15 weeks-old WT, SOD1 and SOD1 treated animals after curative intravenous delivery of AAVPHP.eB-CYP46A1. (B-C) Scoring of NMJ according to their state of innervation and their integrity at 15 weeks of age.

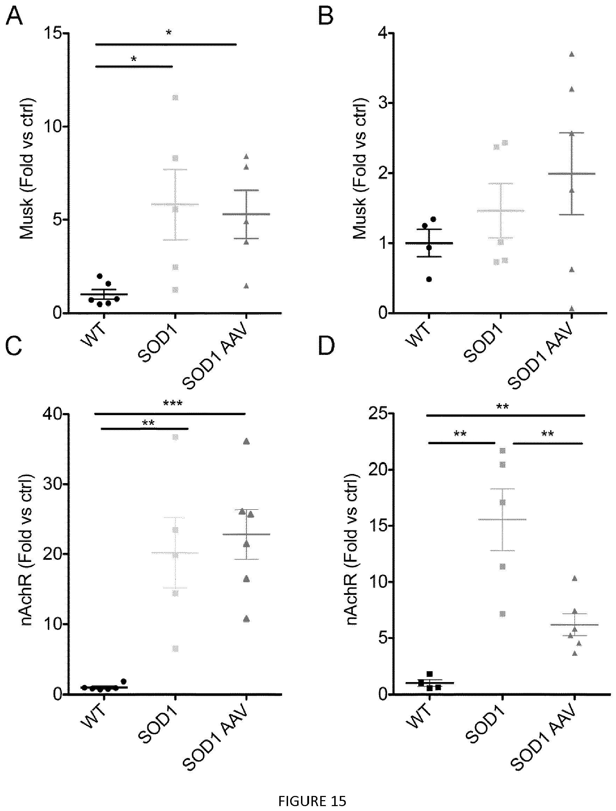

Figure 15 - **Molecular analysis of NMJ at 15 weeks in curative treatment.** Analysis of Musk expression in tibialis (A) and gastrocnemius (B) and nAchR expression quantification in tibialis (C) and gastrocnemius (D).

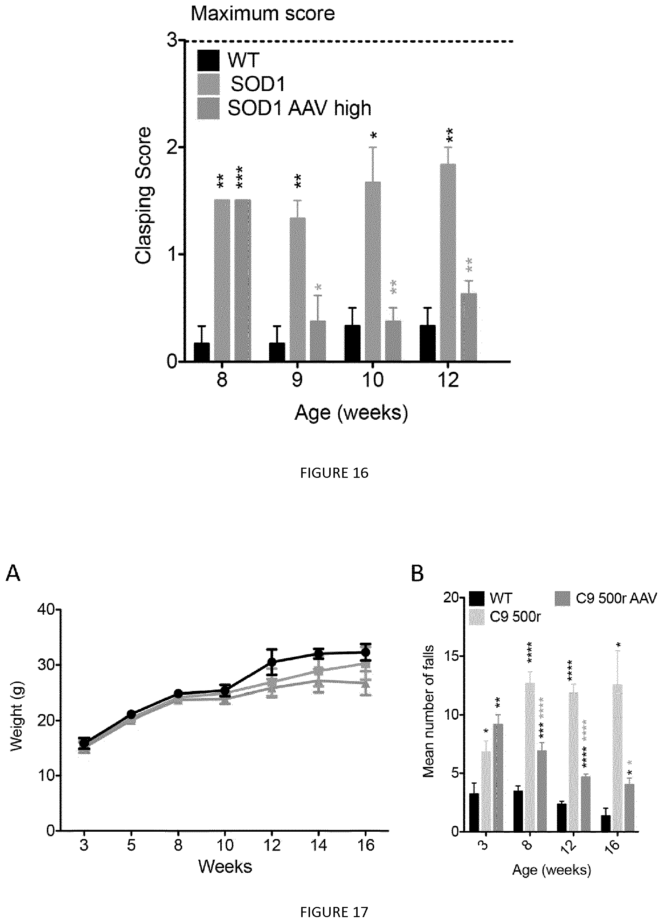

Figure 16 - **Behavioral evaluation of SOD1 mice after intravenous administration of AAVPHP.eB- CYP46A1 at curative stage (8 weeks) at high dose.** Clasping test. Results are presented as mean ± SEM. ^{∗}p<0.05, ^{∗∗}p<0.01, ^{∗∗∗}p<0.001. Black ^{∗} corresponds to p value vs WT animals and grey to SOD1 animals.
Figure 17 **- Behavioral evaluation of C9 ORF72 mice after intravenous administration of AAVPHP.eB- CYP46A1 at preventive stage (4 weeks) at high dose.** (A) Weight follow up and (B) Notched bar performances. Results are presented as mean ± SEM. ^{∗}p<0.05, ^{∗∗}p<0.01, ^{∗∗∗}p<0.001. Black ^{∗} corresponds to p value vs WT animals and grey to C9ORF72 animals.

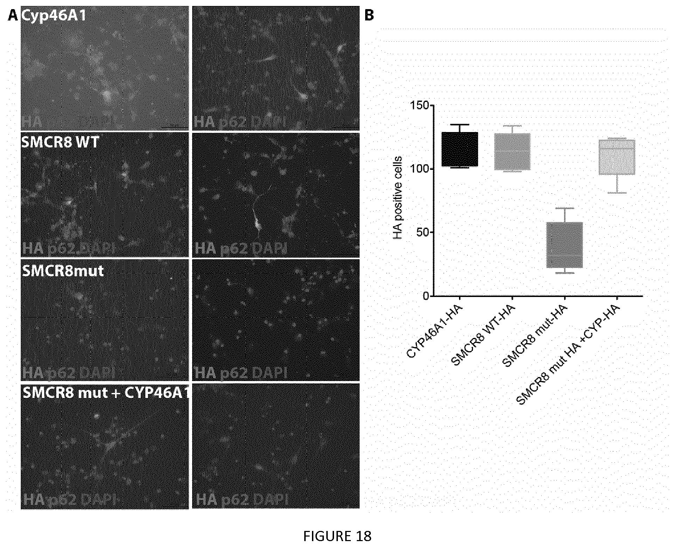

Figure 18 - (A) Evaluation of p62 aggregates in cells after overexpression of SMCR8 WT or mutant and overexpression of CYP46A1. (B) quantification of total number of transduced cells (HA) positive reflecting neuronal survival in the different conditions.

### Detailed description of the invention

The inventors demonstrated that delivering a vector expressing a CYP46A1 gene intravenously in a mouse model of ALS is able to prevent/correct the development of motor impairment. More particularly, the inventors demonstrated that delivering a plasmid expressing a CYP46A1 gene into primary striatal neurons modelling amyotrophic lateral sclerosis pathology, results in a significant decrease of p62 aggregates, which is a hallmark of SMCR8 dysfunction and is involved in ALS through impairment of autophagy and improve survival of neurons.

On this basis, the inventors provide a viral vector for the treatment of ALS, wherein the vector expresses CYP46A1 in cells of the central nervous system, especially motoneurons and neurons within motor cortex. This strategy is useful in treating any motoneurons disorders which are a continuum with some ALS form mainly through common mutated genes. Particularly, the inventors provide a viral vector for the treatment of frontotemporal dementia (FTD) associated to ALS.

### Amyotrophic lateral sclerosis

The present invention specifically relates to treatment of amyotrophic lateral sclerosis. Preferably, the present invention relates to treatment of ALS which are caused by sporadic factor(s) and/or when a disease-associated protein (i.e., SOD1, FUS, C9ORF72, TDP43...) contains a gain of function mutation. In an embodiment, the present invention relates to treatment of ALS associated with at least one motoneuron related disorder. Such related motoneuron disorders are preferably selected from lower motoneuron disorders or upper motoneuron disorders including spastic paraplegia. In a particular embodiment, the present invention relates to treatment of ALS associated with frontotemporal dementia (FTD). FTD is a fatal neurodegenerative disease characterized by neuronal degeneration in the frontal and temporal lobes causing progressive deterioration of language, personality and behavior. At least 15-20% of ALS patients receive concomitant diagnosis of FTD (Prudencio et al. 2015). In another particular embodiment, the present invention relates to treatment of ALS without frontotemporal dementia.

In the context of the invention, the terms "treatment", "treat" or "treating" are used herein to characterize a therapeutic method or process that is aimed at (1) slowing down or stopping the progression, aggravation, or deterioration of the symptoms of the disease state or condition to which such term applies; (2) alleviating or bringing about ameliorations of the symptoms of the disease state or condition to which such term applies; and/or (3) reversing or curing the disease state or condition to which such term applies.

As used herein, the term "subject" or "patient" refers to an animal, preferably to a mammal, even more preferably to a human, including adult and child. However, the term "subject" can also refer to non-human animals, mammals such as mouse, and non-human primates.

### The CYP46A1 sequences

A first object of the invention relates to a vector for use in the treatment of amyotrophic lateral sclerosis, which comprises the full sequence of cholesterol 24-hydroxylase encoding nucleic acid.

As used herein, the term "gene" refers to a polynucleotide containing at least one open reading frame that can encode a particular polypeptide or protein after being transcribed or translated.

As used herein, the terms "coding sequence" or "a sequence which encodes a particular protein", denotes a nucleic acid sequence which is transcribed (in the case of DNA) and translated (in the case of mRNA) into a polypeptide in vitro or in vivo when placed under the control of appropriate regulatory sequences. The boundaries of the coding sequence are determined by a start codon at the 5' (amino) terminus and a translation stop codon at the 3' (carboxy) terminus. A coding sequence can include, but is not limited to, cDNA from prokaryotic or eukaryotic mRNA, genomic DNA sequences from prokaryotic or eukaryotic DNA, and even synthetic DNA sequences.

The CYP46A1 gene encodes cholesterol 24-hydroxylase. This enzyme is a member of the cytochrome P450 superfamily of enzymes. The enzyme converts cholesterol into 24S-hydroxycholesterol (24S-OH-Chol) that can dynamically cross the BBB, accomplishing peripheral circulation to be evacuated out of the body (Björkhem et al. 1998), thus maintaining cholesterol homeostasis. A cDNA sequence for CYP46A1 is disclosed in Genbank Access Number AF094480 (SEQ ID NO:1). The amino acid sequence is shown in SEQ ID NO:2.

The invention makes use of a nucleic acid construct comprising sequence SEQ ID NO:1 or a variant thereof for the treatment of amyotrophic lateral sclerosis, and optionally related motoneuron disorders such as FTD.

The variants include, for instance, naturally-occurring variants due to allelic variations between individuals (e.g., polymorphisms), alternative splicing forms, etc. The term variant also includes CYP46A1 gene sequences from other sources or organisms. Variants are preferably substantially homologous to SEQ ID NO:1, i.e., exhibit a nucleotide sequence identity of typically at least about 75%, preferably at least about 85%, more preferably at least about 90%, more preferably at least about 95% with SEQ ID NO:1. Variants of a CYP46A1 gene also include nucleic acid sequences, which hybridize to a sequence as defined above (or a complementary strand thereof) under stringent hybridization conditions. Typical stringent hybridisation conditions include temperatures above 30° C, preferably above 35°C, more preferably in excess of 42°C, and/or salinity of less than about 500 mM, preferably less than 200 mM. Hybridization conditions may be adjusted by the skilled person by modifying the temperature, salinity and/or the concentration of other reagents such as SDS, SSC, etc.

### Non-viral vectors

In an embodiment, the vector use according to the present invention is a non-viral vector. Typically, the non-viral vector may be a plasmid encoding CYP46A1. This plasmid can be administered directly or through a liposome, an exosome or a nanoparticle.

### Viral vectors

Gene delivery viral vectors useful in the practice of the present invention can be constructed utilizing methodologies well known in the art of molecular biology. Typically, viral vectors carrying transgenes are assembled from polynucleotides encoding the transgene, suitable regulatory elements and elements necessary for production of viral proteins which mediate cell transduction.

The terms "gene transfer" or "gene delivery" refer to methods or systems for reliably inserting foreign DNA into host cells. Such methods can result in transient expression of non-integrated transferred DNA, extrachromosomal replication and expression of transferred replicons (e. g., episomes), or integration of transferred genetic material into the genomic DNA of host cells.

Examples of viral vector include adenovirus, lentivirus, retrovirus, herpes-virus and Adeno-Associated virus (AAV) vectors.

Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO95/14785, WO96/22378, US5,882,877, US6,013,516, US4,861,719, US5,278,056 and WO94/19478.

In a preferred embodiment, adeno-associated viral (AAV) vectors are employed.

By an "AAV vector" is meant a vector derived from an adeno-associated virus serotype, including without limitation, AAV1, AAV2, AAV3, AAV4, AAV5, AAV6, AAV8, AAV9, AAV10, such as AAVrh.10, AAVPHP.eB, etc. AAV vectors can have one or more of the AAV wild-type genes deleted in whole or part, preferably the rep and/or cap genes, but retain functional flanking ITR sequences. Functional ITR sequences are necessary for the rescue, replication and packaging of the AAV virion. Thus, an AAV vector is defined herein to include at least those sequences required in cis for replication and packaging (e. g., functional ITRs) of the virus. The ITRs need not be the wild-type nucleotide sequences, and may be altered, e. g, by the insertion, deletion or substitution of nucleotides, so long as the sequences provide for functional rescue, replication and packaging. AAV expression vectors are constructed using known techniques to at least provide as operatively linked components in the direction of transcription, control elements including a transcriptional initiation region, the DNA of interest (i.e. the CYP46A1 gene) and a transcriptional termination region. Two copies of the DNA of interest can be included, as a self-complementary construct.

In a more preferred embodiment, the AAV vector is an AAV9, AAV10, preferably AAVrh.10, AAVPHP.B or AAVPHP.eB vector, or vector derived from one of these serotypes. In a most preferred embodiment, the AAV vector is an AAVPHP.eB vector. The AAVPHP.eB vector is evolved AAV-PHP.B variant that efficiently transduces CNS neurons (Chan KY et al. 2017 ; WO2017100671). Other vectors, such as the ones described in WO2015038958 and WO2015191508 may also be used.

The control elements are selected to be functional in a mammalian cell. The resulting construct which contains the operatively linked components is bounded (5' and 3') with functional AAV ITR sequences. By "adeno-associated virus inverted terminal repeats " or "AAV ITRs" is meant the art-recognized regions found at each end of the AAV genome which function together in cis as origins of DNA replication and as packaging signals for the virus. AAV ITRs, together with the AAV rep coding region, provide for the efficient excision and rescue from, and integration of a nucleotide sequence interposed between two flanking ITRs into a mammalian cell genome. The nucleotide sequences of AAV ITR regions are known. (See, e. g., Kotin, 1994; Berns, KI "Parvoviridae and their Replication" in Fundamental Virology, 2nd Edition, (B. N. Fields and D. M. Knipe, eds.) for the AAV-2 sequence. As used herein, an "AAV ITR" does not necessarily comprise the wild-type nucleotide sequence, but may be altered, e. g., by the insertion, deletion or substitution of nucleotides. Additionally, the AAV ITR may be derived from any of several AAV serotypes, including without limitation, AAV1, AAV2, AAV3, AAV4, AAV5, AAV6, etc. Furthermore, 5' and 3' ITRs which flank a selected nucleotide sequence in an AAV vector need not necessarily be identical or derived from the same AAV serotype or isolate, so long as they function as intended, i.e., to allow for excision and rescue of the sequence of interest from a host cell genome or vector, and to allow integration of the heterologous sequence into the recipient cell genome when AAV Rep gene products are present in the cell.

Particularly preferred are vectors derived from AAV serotypes having tropism for and high transduction efficiencies in cells of the mammalian central nervous system (CNS), particularly neurons. A review and comparison of transduction efficiencies of different serotypes is provided in Davidson et al., 2000. In one preferred example, AAV2 based vectors have been shown to direct long-term expression of transgenes in CNS, preferably transducing neurons. In other non-limiting examples, preferred vectors include vectors derived from AAV4 and AAV5 serotypes, which have also been shown to transduce cells of the CNS (Davidson et al, supra). In particular, the vector may be an AAV vector comprising a genome derived from AAV5 (in particular the ITRs are AAV5 ITRs) and a capsid derived from AAV5.

In a particular embodiment of the invention, the vector is a pseudotyped AAV vector. Specifically, a pseudotyped AAV vector comprises an AAV genome derived from a first AAV serotype and a capsid derived from a second AAV serotype. Preferably, the genome of the AAV vector is derived from AAV2. Furthermore, the capsid is preferably derived from AAV5. Specific non-limiting examples of pseudotyped AAV vectors include an AAV vector comprising a genome derived from AAV2 in a capsid derived from AAV5, an AAV vector comprising a genome derived from AAV2 in a capsid derived from AAVrh.10, etc.

The selected nucleotide sequence is operably linked to control elements that direct the transcription or expression thereof in the subject in vivo. Such control elements can comprise control sequences normally associated with the selected gene. In particular, such control elements may include the promoter of the CYP46A1 gene, in particular the promoter of the human CYP46A1 gene (Ohyama Y et al., 2006)

Alternatively, heterologous control sequences can be employed. Useful heterologous control sequences generally include those derived from sequences encoding mammalian or viral genes. Examples include, but are not limited to, the phophoglycerate kinase (PGK) promoter, the SV40 early promoter, mouse mammary tumor virus LTR promoter; adenovirus major late promoter (Ad MLP); a herpes simplex virus (HSV) promoter, a cytomegalovirus (CMV) promoter such as the CMV immediate early promoter region (CMVIE), rous sarcoma virus (RSV) promoter, synthetic promoters, hybrid promoters, and the like. In addition, sequences derived from nonviral genes, such as the murine metallothionein gene, will also find use herein. Such promoter sequences are commercially available from, e. g., Stratagene (San Diego, CA). For purposes of the present invention, both heterologous promoters and other control elements, such as CNS-specific and inducible promoters, enhancers and the like, will be of particular use.

Examples of heterologous promoters include the CMV promoter. Examples of CNS specific promoters include those isolated from the genes from myelin basic protein (MBP), glial fibrillary acid protein (GFAP), synapsins (e.g. human sysnapsin 1 gene promoter), and neuron specific enolase (NSE).

Examples of inducible promoters include DNA responsive elements for ecdysone, tetracycline, hypoxia andaufin.

The AAV expression vector which harbors the DNA molecule of interest bounded by AAV ITRs, can be constructed by directly inserting the selected sequence(s) into an AAV genome which has had the major AAV open reading frames ("ORFs") excised therefrom. Other portions of the AAV genome can also be deleted, so long as a sufficient portion of the ITRs remain to allow for replication and packaging functions. Such constructs can be designed using techniques well known in the art. See, e. g., U. S. Patents Nos. 5,173,414 and 5,139,941; International Publications Nos. WO 92/01070 (published 23 January 1992) and WO 93/03769 (published 4 March 1993); Lebkowski et al., 1988 ; Vincent et al., 1990; Carter, 1992 ; Muzyczka, 1992 ; Kotin,1994; Shelling and Smith, 1994 ; and Zhou et al., 1994. Alternatively, AAV ITRs can be excised from the viral genome or from an AAV vector containing the same and fused 5' and 3' of a selected nucleic acid construct that is present in another vector using standard ligation techniques. AAV vectors which contain ITRs have been described in, e. g.,

US. Patent no. 5,139,941. In particular, several AAV vectors are described therein which are available from the American Type Culture Collection ("ATCC") under Accession Numbers 53222, 53223, 53224, 53225 and 53226. Additionally, chimeric genes can be produced synthetically to include AAV ITR sequences arranged 5' and 3' of one or more selected nucleic acid sequences. Preferred codons for expression of the chimeric gene sequence in mammalian CNS cells can be used. The complete chimeric sequence is assembled from overlapping oligonucleotides prepared by standard methods. (See, e. g., Edge, 1981 ; Nambair et al., 1984 ; Jay et al., 1984). In order to produce rAAV virions, an AAV expression vector is introduced into a suitable host cell using known techniques, such as by transfection. A number of transfection techniques are generally known in the art. (See, e.g., Graham et al., 1973; Sambrook et al. (1989) Molecular Cloning, a laboratory manual, Cold Spring Harbor Laboratories, New York, Davis et al. (1986) Basic Methods in Molecular Biology, Elsevier, and Chu et al., 1981). Particularly suitable transfection methods include calcium phosphate coprecipitation (Graham et al., 1973), direct microinjection into cultured cells (Capecchi, 1980), electroporation (Shigekawa et al., 1988), liposome mediated gene transfer (Mannino et al., 1988), lipid-mediated transduction (Felgner et al., 1987), and nucleic acid delivery using high-velocity microprojectiles (Klein et al., 1987).

For instance, a preferred viral vector, such as the AAVPHP.eB, comprises, in addition to a cholesterol 24-hydroxylase encoding nucleic acid sequence, the backbone of AAV vector with ITR derived from AAV-2, the promoter, such as the mouse PGK (phosphoglycerate kinase) gene or the cytomegalovirus/β-actin hybrid promoter (CAG) consisting of the enhancer from the cytomegalovirus immediate gene, the promoter, splice donor and intron from the chicken β-actin gene, the splice acceptor from rabbit β-globin, or any neuronal promoter such as the promoter of Dopamine-1 receptor or Dopamine-2 receptor with or without the wild-type or mutant form of woodchuck hepatitis virus post-transcriptional regulatory element (WPRE).

### Delivery of the vectors

A method of treatment of amyotrophic lateral sclerosis is disclosed, which method comprises administering a vector comprising cholesterol 24-hydroxylase encoding nucleic acid to a patient in need thereof. The vector may be delivered directly into the brain and/or spinal cord of the subject or by intravascular, intravenous, intranasal, intraventricular or intrathecal injection. In a particular embodiment, the vector is AAVrh10 or AAVPHP.eB and is delivered by intravenous injection.

In a particular embodiment, a method for treating amyotrophic lateral sclerosis (ALS) in a subject, is disclosed, said method comprising:
(a) providing a vector as defined above, which comprises a cholesterol 24-hydroxylase encoding nucleic acid; and
(b) delivering the vector to the brain and/or spinal cord of the subject, whereby said vector transduces cells in the brain and/or spinal cord, and whereby cholesterol 24-hydroxylase is expressed by the transduced cells at a therapeutically effective level.

Advantageously, the vector is a viral vector, more advantageously an AAV vector, even advantageously an AAV vector selected from the group consisting of AAV9, AAV10, or AAVPHP.eB.

In a particular embodiment, the vector is delivered to brain, particularly to motor cortex, and/or spinal cord. In a particular embodiment, the vector is delivered exclusively to spinal cord.

In another particular embodiment, the vector is administered by intravenous injection.

Methods of delivery, or administration, of viral vectors to neurons and/or astrocytes and/or oligodendrocytes and/or microglia include generally any method suitable for delivery vectors to said cells, directly or through hematopoietic cells transduction, such that at least a portion of cells of a selected synaptically connected cell population is transduced. The vector may be delivered to any cells of the central nervous system, cells of the peripheral nervous system, or both. Preferably, the vector is delivered to cells of the brain and/or spinal cord. Generally, the vector is delivered to the cells of the brain, including for example cells of motor cortex, spinal cord or combinations thereof, or any suitable subpopulation thereof.

To deliver the vector specifically to a particular region and to a particular population of cells of the brain or the spinal cord, the vector may be administered by stereotaxic microinjection. For example, patients have the stereotactic frame base fixed in place (screwed into the skull). The brain with stereotactic frame base (MRI compatible with fiducial markings) is imaged using high resolution MRI. The MRI images are then transferred to a computer which runs stereotactic software. A series of coronal, sagittal and axial images are used to determine the target (site of vector injection) and trajectory. The software directly translates the trajectory into 3 dimensional coordinates appropriate for the stereotactic frame. Burr holes are drilled above the entry site and the stereotactic apparatus positioned with the needle implanted at the given depth. The vector is then injected at the target sites, eventually mixed with a contrast agent. Since the vector integrates into the target cells, rather than producing viral particles, the subsequent spread of the vector is minor, and mainly a function of passive diffusion from the site of injection and of course the desired trans-synaptic transport, prior to integration. The degree of diffusion may be controlled by adjusting the ratio of vector to fluid carrier.

Additional routes of administration may also comprise local application of the vector under direct visualization, e. g., superficial cortical application, intranasal application, or other non-stereotactic application.

The target cells of the vectors of the present invention are cells of the spinal cord (motoneurons) and of the brain (motor cortex) of a subject afflicted with ALS, preferably neural cells.

Preferably the subject is a human being, generally an adult but may be a child or an infant. However, the invention encompasses delivering the vector to biological models of the disease. In that case, the biological model may be any mammal at any stage of development at the time of delivery, e. g., embryonic, foetal, infantile, juvenile or adult, preferably it is an adult. Furthermore, the target cells may be essentially from any source, especially nonhuman primates and mammals of the orders Rodenta (mice, rats, rabbit, hamsters), Carnivora (cats, dogs), and Arteriodactyla (cows, pigs, sheep, goats, horses) as well as any other non-human system (e. g. zebrafish model system).

Preferably, the use of the invention comprises intracerebral administration, through stereotaxic injections. However, other known delivery methods may also be adapted in accordance with the invention. For example, for a more widespread distribution of the vector across the brain, it may be injected into the cerebrospinal fluid, e. g., by lumbar puncture, cisterna magna or ventricular puncture. To direct the vector to the brain, it may be injected into the spinal cord or into the peripheral ganglia, or the flesh (subcutaneously or intramuscularly) of the body part of interest. In certain situations, such as here with AAVPHP.eB, the vector can be administered via an intravascular approach. For example, the vector can be administered intra-arterially (carotid) in situations where the blood-brain barrier is disturbed. Moreover, for more global delivery, the vector can be administered during the "opening" of the blood-brain barrier achieved by infusion of hypertonic solutions including mannitol or ultra-sound local delivery.

The vectors used herein may be formulated in any suitable vehicle for delivery. For instance, they may be placed into a pharmaceutically acceptable suspension, solution or emulsion. Suitable mediums include saline and liposomal preparations. More specifically, pharmaceutically acceptable carriers may include sterile aqueous of non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Intravenous vehicles include fluid and nutrient replenishers, electrolyte replenishers (such as those based on Ringer's dextrose), and the like.

Preservatives and other additives may also be present such as, for example, antimicrobials, antioxidants, chelating agents, and inert gases and the like.

A colloidal dispersion system may also be used for targeted gene delivery. Colloidal dispersion systems include macromolecule complexes, nanocapsules, microspheres, beads, and lipid-based systems including oil-in-water emulsions, micelles, mixed micelles, and liposomes or exosomes.

The preferred doses and regimen may be determined by a physician, and depend on the age, sex, weight, of the subject, and the stage of the disease. As an example, for delivery of cholesterol 24-hydroxylase using a viral expression vector, each unit dosage of cholesterol 24-hydroxylase expressing vector may comprise 2.5 to 100 µl of a composition including a viral expression vector in a pharmaceutically acceptable fluid and which provides from 1010 up to 1015 cholesterol 24-hydroxylase expressing viral particles per ml of composition.

The vector may be used in curative treatment and/or in preventive treatment.

It is thus an object of the present invention to provide a vector which comprises cholesterol 24-hydroxylase encoding nucleic acid, for use in a preventive treatment of ALS.

It is another object of the present invention to provide a vector which comprises cholesterol 24-hydroxylase encoding nucleic acid, for use in a curative treatment of ALS.

### Pharmaceutical composition

A further object of the invention concerns a pharmaceutical composition for use in the treatment of ALS, which comprises a therapeutically effective amount of a vector according to the invention.

By a "therapeutically effective amount" is meant a sufficient amount of the vector of the invention to treat ALS at a reasonable benefit/risk ratio applicable to any medical treatment.

It will be understood that the total daily dosage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range per adult per day. The therapeutically effective amount of the vector according to the invention that should be administered, as well as the dosage for the treatment of a pathological condition with the number of viral or non-viral particles and/or pharmaceutical compositions of the invention, will depend on numerous factors, including the age and condition of the patient, the severity of the disturbance or disorder, the method and frequency of administration and the particular peptide to be used.

The presentation of the pharmaceutical compositions that contain the vector according to the invention may be in any form that is suitable for intramuscular, intracerebral, intranasal, intrathecal, intraventricular or intravenous administration. In the pharmaceutical compositions of the present invention for intramuscular, intranasal, intravenous, intracerebral, intrathecal or intraventricular administration, the active principle, alone or in combination with another active principle, can be administered in a unit administration form, as a mixture with conventional pharmaceutical supports, to animals and human beings.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of sterile injectable solutions.

Upon formulation, solutions will be administered in a manner compatible with the dosage formulation and in such amount as is therapeutically effective. The formulations are easily administered in a variety of dosage forms, such as the type of injectable solutions, but drug release capsules and the like can also be employed.

Multiple doses can also be administered.

It is also possible to associate the therapeutical approach with small molecules that could activate CYP46A1 such as efavirenz (Mast N et al. 2013 ; Mast N et al. 2017 ; Mast N et al. 204 ; Mast N et al. 2017) or antifungal drugs (Mast N et al. 2013 ; Fourgeux et al. 2014) which on the contrary could inhibit CYP46A1 and thus be a way to stop the gene therapy approach if needed.

The invention will be further illustrated by the following example. However, this example and the accompanying figures should not be interpreted in any way as limiting the scope of the present invention.

### EXAMPLE

In this experiment, an in vitro model of ALS has been generated, based on overexpression of SMCR8 D928V, a mutant of SMCR8, which form a complex with C9ORF72 and WDR1 and act as a GDP/GTP exchange factor for RAB8 and RAB39b and thus control autophagic flux (Sellier et al. 2016). Either the WT SMCR8 or the mutant SMCR8, which alter autophagy as expression of siRNA for SMCR8, have been overexpressed.

### Materials and Methods

### Animals

Two males B6SJL-Tg(SOD1^{∗}G93A)1Gur/J were obtained from Jackson Laboratories (stock 002726) and mated with C57BL/6 mice to generate the colony.

Three males FVB/NJ-Tg(C9orf72)500Lpwr/J were obtained from Jackson Laboratories (stock 029099) and mated with FVB/NJ mice to generate the colony.

Mice were housed in a temperature-controlled room and maintained on a 12 h light/dark cycle. Food and water were available ad libitum. The experiments were carried out in accordance with the European Community Council directive (2010/63/EU) for the care and use of laboratory animals.

### AAV plasmid design and vector production

AAV vectors were produced and purified by Atlantic Gene therapies (INSERM U1089, Nantes, France). Vector production has been described elsewhere (Hudry E et al. 2010). The viral constructs for AAVPHP.eB-CYP46A1-HA contained the expression cassette consisting of the human Cyp46algenes, driven by a CMV early enhancer/chicken β-actin (CAG) synthetic promoter (CAG) surrounded by inverted terminal repeats (ITR) sequences of AAV2. The final titers of the batches were between 1.5 and 2.10¹³vg/ml.

### Genotyping

Mice were genotype according to the Jackson lab procedure (https://www2.jax.org/protocolsdb/f?p=116:5:0::NO:S:P5_MASTER_PROTOCOL_ID,P5_J RS_CODE:24173,002726) for SOD1G93A mouse line and https://www2.jax.org/protocolsdb/f?p=116:5:0::NO:5:P5_MASTER_PROTOCOL_ID,P5_JR S_CODE:30889,029099 for C9ORF72 500r line.

### Intravenous injection

In order to determine the dose of AAVPHP.eB- CYP46A1-HA to inject in ALS mouse model, C57BL6 animals have been injected with 3 doses 2.5.10¹¹vg total (low), 5.10¹¹vg total (medium) and 1.10¹²vg total (high dose) (n=3 per group) at the age of 8 weeks.

For preventive treatment, three-week-old SOD1^{G93A} or four-week-old C9ORF72 500r mice were induced for anesthesia with isoflurane at 4% and then maintained at 2% with 80% air and 20% oxygen. SOD1 animal received an injection of 2.5.1011vg total (low dose) or 5.1011vg total (medium dose) (100µl volume) intravenously by retroorbital injection. WT and non-injected SOD1 animals received injection of 100µl of saline solution.

Details of the preventive groups:
- SOD1 study: n=10 WT; n=12 SOD1 and n=14 SOD1 AAV at 2.5.10¹¹vg total
- C9 study: n=16 WT; n=16 C9 and n=17 C9 AAV at 5.10¹¹vg total

For curative treatment, eight-week-old SOD1G93A or sixteen-week-old C9ORF72 500r mice were induced for anesthesia with isoflurane at 4% and then maintained at 2% with 80% air and 20% oxygen. SOD1 animal received an injection of 2.5.10¹¹vg total (low dose) or 5.10¹¹vg total (medium dose) (100µl volume) intravenously by retroorbital injection. WT and non-injected SOD1 animals received injection of 100ul of saline solution.

Details of the curative groups:
- SOD1 study:
   o n=24 WT; n=27 SOD1 and n=22 SOD1 AAV at 2.5.10¹¹vg total
   o n=5 WT; n=5 SOD1 and n=7 SOD1 AAV at 5.10¹¹vg total
- C9 study: n=14 WT; n=13 C9 and n=12 C9 AAV at 5.10¹¹vg total

### Behavioral tests

### Weight follow up

All animals were weighted prior to injection and each weeks for SOD1 animals and each two weeks for C9ORF72 animals.

### Clasping test

Clasping test evaluates coordination and is classical for ALS evaluation. Animals were scored prior to injection and then each 2 weeks from 6 or 8 weeks until 15 weeks. Animals are maintained from the tail and the score is one if mice are twitching, and then a point is added to the score for each hindlimbs clasping. Results are presented for each test as mean ± SEM for each group and t-test analyses were performed.

### Inverted test

Inverted test evaluate strength and is classical for ALS evaluation. Animals were scored prior to injection and then each 2 weeks from 6 or 8 weeks until 15 weeks. Animals are placed on the grid and grid is inverted, immediate soring of remaining feet attached on the grid is done (short inverted). Results are presented for each test as mean ± SEM for each group and t-test analyses were performed.

For C9 model, mice were challenged even more for inverted test to have more robust results by 3 trials of 5 minutes each with 15 min recovery between each trial. For each trial, the time that they maintain attached is scored and the mean is calculated for each animal.

### Notched bar

Coordination was evaluated using the notched-bar test (scored number of falls of the upper or lower limbs) as described previously (Piguet F et al. 2018).

### Survival evaluation

Mice were observed daily and euthanized immediately if they met endpoint criteria (dragged hindlimbs or ≥20% loss of body weight).

### Tissue collection

Mice were anesthetized with pentobarbital (Euthasol 180 mg/kg) solution and perfused transcardially with phosphate buffered saline (PBS). Brain, spinal cord and sciatic nerves as well as peripheral organs (liver, heart, lung, kidney, spleen) were collected and post-fixed in PFA 4% prior to paraffin inclusion for histology (Cut 6-10 µm with microtome) or immediately frozen in liquid nitrogen for biomolecular analysis.

Limbs muscles were dissected and post-fixed overnight at 4°C in 4% PFA/PBS. Samples were rinsed three times in PBS and cryoprotected in 20% sucrose/PBS for 48 hours. Tissues were embedded in cryomatrix (Thermoscientific) and cut (20 µm) using a cryostat (Leica CM3050S). Cryosections were dried at room temperature and stored at -20°C.

Gastrocnemium, tibialis, quadriceps and a part of spinal cord were dissected and were frozen in liquid azote.

Different tissues were grinded/crushed in liquid azote and were separated to analyze RNA or DNA expression.

### Primary striatal cell culture and transfection

Primary striatal neurons were dissected from Day 14 embryos from pregnant Swiss mice (Janvier) as previously described (Charvin D et al. 2005 ; Deyts et al. 2009). After 7 days in vitro, transient transfection of striatal cells was performed with LipofectamineTM 2000 (Invitrogen). At this stage, very few glial cells were observed (55%; data not shown). Cells were transfected following 5 conditions (CYP46A1-HA; SMWR8 WT-HA; SMCR8 D928V-HA, SMWR8 WT-HA + CYP46A1-HA; SMCR8 D928V-HA + CYP46A1HA) and non-transfected cells were used as controls. N=6 replicates were performed for each condition. Transfection with Lipofectamine in 8 wells labtek chambers with 100ng of DNA for each plasmid for 16h prior to cell fixation.

### Primary antibodies

Antibodies used immunohistochemical (IHC) analyses are listed in Table 1, below.

**Table 1: Antibodies used in immunofluorescence and immunohistochemical (IHC) analyses**

| **Primary antibodies** | **Source** | **IF** |
|---|---|---|
| mouse anti-HA | Biolegend (901514) | 1:1000 |
| mouse anti-p62 | Abcam (ab56416) | 1:250 |
| Rabbit anti-HA | Cell signaling (#3724) | 1:500 |
| Rabbit anti-Iba1 | Wako (019-19741) | 1:500 |
| Mouse anti-GFAP | Sigma (G-3893) | 1:500 |
| Mouse anti-Pan-NF | Biolegend (837904) | 1:1000 |
| Alexa 488- αBTX | Lifesciences (B13422) | 1:2000 |
| Rabbit anti-VachT | Sigma (SABA2000559) | 1:1000 |

### Immunostaining

### On cells

The immunofluorescence procedure was initiated, by fixation of cells for 15min in PFA. After three washes, cells were permeabilized in PBS/0.3% Triton X-100 and then blocked in PBS/0.1% Triton X-100 containing 5% Normal Goat Serum (NGS, Gibco) for 1h at RT. The cells were then incubated with the respective primary antibodies, overnight at 4°C. After three washes, the cells were incubated with the corresponding secondary antibody (1:1000; Vector Laboratories Inc., CA, USA) diluted in PBS/0.1% Triton X-100 for 1h at RT. After three washes, the cells were dry and mounted in mounting medium with DAPI.

### On paraffin sections

Muscles: Muscle cryosections were treated with 0.1 M glycine in PBS for 30 min before processing. After washes in PBS, sections were permeabilized with PBS/0.3% TritonX-100 for 10 min and saturated with PBS/0.3% Triton/ 10% BSA for 45 min. The primary antibodies were diluted in the saturation solution and incubated O/N at 4°C. After washes in PBS/0.1% Triton, the secondary antibodies and α-bungarotoxin were diluted in PBS/0.1% Triton/10% BSA and added for 1 hour at room temperature. After washes in PBS/0.1% Triton, the slides were mounted with fluorescent aqueous mounting medium (F4680, Sigma).

Primary antibodies for immunofluorescence were the mouse anti Pan-Neurofilament (1:1000; Biolegend, 837904), rabbit anti vesicular acetylcholine transporter (VAChT; 1:1000 ; sigma, 2000559). Secondary antibodies were diluted 1:1000 and were donkey anti-rabbit/AlexaFluor 594, anti-mouse/AlexaFluor Cy3 (Life Technologies, Carlsbad, CA, USA).

Alpha-bungarotoxin-Alexa594 (1:2000; Life Technologies, B13422) was incubated with the secondary antibodies. Pictures were taken with a confocal SP8 Leica DLS inverted microscope (Carl Zeiss, Zaventem, Belgium). For all images, brightness and contrast were adjusted with ImageJ software after acquisition to match with the observation.

Spinal cord: For immunofluorescence, spinal cords were treated 10mM Tris/1mM EDTA/0,1% Tween pH8,75 at 95°C for 45 min. After washes in PBS, sections were incubated with the permeabilization solution (PBS/0.3% TritonX-100) for 15 min and then with saturation solution (PBS/0,1%TritonX-100/10% horse serum) for 1 hour. The primary antibodies were diluted in 10% horse serum/PBS/0,1%Triton X-100 and incubated on tissue sections overnight at 4°C. After washes in PBS/0,1% TritonX-100, sections were incubated with secondary antibodies (donkey anti-rabbit Alexa 594 and donkey anti-mouse alexa 488, 1 :1000, Life Technologies).

The immunohistochemical labeling was performed using the ABC method. Briefly, tissue sections were treated with peroxide for 30 minutes to inhibit endogenous peroxidase. After washes in PBS, sections were treated 10mM Tris/1mM EDTA/0,1% Tween pH8,75 at 95°C for 45 min (Only for anti-HA). After washes in PBS, sections were incubated with the blocking solution (10% goat serum or goat serum in PBS/0,3% TritonX-100) for 1 hour. The primary antibodies were diluted in blocking solution and incubated on tissue sections overnight at 4°C. After washes in PBS, sections were sequentially incubated with goat anti-rabbit or goat anti-mouse antibodies conjugated to biotin (Vector Laboratories) for 30 minutes at room temperature, followed by the ABC complex (Vector Laboratories). After washes in PBS, the peroxidase activity was detected using diaminobenzidine as chromogene (Dako, Carpinteria, CA). In some case, the slides were counterstained with hematoxylin. The slides were mounted with Depex (VWR International).

### Luxol Staining

Myelin on spinal cord sections was stained/visualized with classical luxol staining.

### Image acquisition

Images of immunofluorescence slides were acquired with a macroscope (Leica) and LAS V3.8 (Leica) software, at room temperature, with a Leica DM 5000B microscope equipped with a Leica DFC310FX digital camera. Photographs for comparison were taken under identical conditions of image acquisition, and all adjustments of brightness and contrast were applied uniformly to all images for cell culture analysis.

For muscle and VachT pictures were acquired with an axioscan, Zeiss with ZEN 2.6 software or nanozoomer. For all images, brightness and contrast were adjusted with ImageJ software after acquisition to match with the observation.

For all IHC and coloration, slices were acquired using the Hamamatsu slide scanner.

### Muscle fiber analysis

Tibialis anterior (TA), gastrocnemius (gastro) and quadriceps (quadri) muscles were dissected at 15 weeks. They were formalin-fixed, paraffin-embedded, cut (10 mm) and stained with hematoxylin/eosin. Pictures were acquired with an axioscan, Zeiss with ZEN 2.6 software. The muscle fiber cross-sectional area (CSA) was measured on minimum 150 fibers for TA, Gastro, Quadri in each animal (n= 4-13) using the ImageJ software.

### Neuromuscular junction analysis

NMJ morphology was quantified on a minimum of 100 TA junctions from 3 different animals. Endplate distribution was classified into six categories: (1) normal endplate, (2) modified endplate, (3) fragmented endplate, (4) degraded endplate, (5) endplate without hole and (6) ectopic endplate. A minimum of 100 junctions was analyzed for each animal (n= 3-4). Motor endplate maturation was evaluated in P21 mice based on criteria previously described (Audouard et al.).

### Motoneuron quantification

The number of motoneurons in cervical, thoracic and lumbar spinal cord sections was quantified after immunohistochemistry for VAChT. The number of transduced motoneurons was quantified after immunohistochemistry for HA. Counting of motoneurons was performed on the left and right ventral sides of three spinal cord sections for each mouse (n = 3).

### Luxol quantification

Total area of myelin has been measured on lumbar spinal cord using Fiji software and normalized by total spinal cord area. The myelin percentage have then been reported as 100% in WT and results are reported compared to WT animals.

### DNA extraction

DNA was extracted from brain, spinal cord and peripheral organs using chloroform/phenol protocol.

### RNA extraction

Total RNA was extracted from a part of lumbar spinal cord, gastrocnemium, tibialis and quadriceps in mice and lumbar spinal cord of patient using Trizol or TriReagent (Sigma). One microgram of total RNA was transcribed into cDNA with Transcriptor First Strand cDNA synthesis kit (Roche) according the manufacturer's instructions.

### q-PCR

cDNA was amplified with SyberGreen (Roche). Primers for RT-qPCR were table above. The amplification protocol for all primers a hot start (95°C for 5 min), 45 amplification cycles (95°C for 15s, 60°C for 1 min) and a melt-curve analysis. Data were analyzed using the Lightcycler 480 software with efficiency factor for each gene and normalized to actine.

| Name | Primer 5' -> 3' |
|---|---|
| *Actine 126 For SEQ ID NO: 3* | TCC TGA GCG CAA GTA CTC TGT |
| *Actine 127 Rev SEQ ID NO:4* | CTG ATC CAC ATC TGC TGG AAG |
| *AchR alpha For SEQ ID NO:5* | AGA TCA TTG TCA CTC ACT TTC CCT |
| *AchR alpha Rev SEQ ID NO:6* | ACG AAG TGG TAG GTG ATG TCC AG |
| *MuSK For SEQ ID NO:7* | TCA TCAC CAC GCC TCT TGA AAC |
| *MuSK Rev SEQ ID NO: 8* | CAT CAT CAC TGT CTT CCA CGC TC |
| *Cyp46A1 mouse For SEQ ID NO:9* | GGC TAA GAA GTA TGG TCC TGT TGT AAG A |
| *cyp46A1 mouse Rev SEQ ID NO:10* | GGT GGA CAT CAG GAA CTT CTT GAC T |
| *ApoE For SEQ ID NO:11* | GTC ACA TTG CTG ACA GGA TGC CTA |
| *ApoE Rev SEQ ID NO:12* | GGG TTG GTT GCT TTG CCA CTC |
| *Hmgcr For SEQ ID NO:13* | CCC CAC ATT CAC TCT TGA CGC TCT |
| *Hmgcr Rev SEQ ID NO:14* | GCT GGC GGA CGC CTG ACA T |
| *Srebp1 For SEQ ID NO:15* | GGT CCA GCA GGT CCC AGT TGT |
| *Srebp1 Rev SEQ ID NO:16* | CTG CAG TCT TCA CGG TGG CTC |
| *Srebp2 For SEQ ID NO:17* | TGT TGA CGC AGA CAG CCA ATG |
| *gadph human for SEQ ID NO:18* | CGC TCT CTG CTC CTC CTG TT |
| *gadph Human Rev SEQ ID NO:19* | CCA TGG TGT CTG AGC GAT GT |
| *cyp46A1 human For SEQ ID NO:20* | CGA GTC CTG AGT CGG TTA AGA AGT T |
| *cyp46A1 human Rev SEQ ID NO:21* | AGT CTG GAG CGC ACG GTA CAT |
| *mADCK3 for SEQ ID NO:22* | CCA CCT CTC CTA TGG GCA GA |
| *mADCK3 rev SEQ ID NO:23* | CCG GGC CTT TTC AAT GTC T |

Vector Genome Copy Number was measured by qPCR on extracted genomic DNA from DRG, spinal cord (cervical, thoracic, and lumbar levels), brain, cerebellum and peripheral organs using the Light Cycler 480 SYBR Green I Master (Roche, France). The results (vector genome copy number per cell) were expressed as n-fold differences in the transgene sequence copy number relative to the Adck3 gene copy as internal standard (number of viral genome copy for 2N genome).

### Cholesterol and oxysterol measurements

Cholesterol and oxysterol analysis followed the 'gold standard' method²⁵ to minimize the formation of autoxidation artefacts. Briefly, mouse striatal tissue samples were weighed and homogenized with a Tissue Lyser II apparatus (Qiagen) in a 500 µl solution containing butylated hydroxytoluene (BHT, 50 µg/ml) and EDTA (0.5 M). At this point, a mix of internal standards was added [epicoprostanol, 2H7-7-lathosterol, 2H6-desmosterol, 2H6-lanosterol and 2H7-24(R/S)-hydroxycholesterol] (Avanti Polar Lipids). Alkaline hydrolysis was performed under Ar using 0.35 M ethanolic KOH for 2 h at room temperature. After neutralization of the solution with phosphoric acid, sterols were extracted in chloroform. The lower phase was collected, dried under a stream of nitrogen and the residue was dissolved in toluene. Oxysterols were then separated from the cholesterol and its precursors on a 100 mg Isolute silica cartridge (Biotage); cholesterol was eluted in 0.5% propan-2-ol in hexane followed by oxysterols in 30% propan-2-ol in hexane. The sterol and oxysterol fractions were independently silylated with Regisil^{®} + 10% TMCS [bis(trimethylsilyl) trifluoro-acetamide + 10% trimethylchlorosilane] (Regis technologies) as described previously²⁶. The trimethylsilylether derivatives of sterols and oxysterols were separated by gas chromatography (Hewlett-Packard 6890 series) in a medium polarity capillary column RTX-65 (65% diphenyl 35% dimethyl polysiloxane, length 30 m, diameter 0.32 mm, film thickness 0.25 µm; Restesk). The mass spectrometer (Agilent 5975 inert XL) in series with the gas chromatography was set up for detection of positive ions. Ions were produced in the electron impact mode at 70 eV. They were identified by the fragmentogram in the scanning mode and quantified by selective monitoring of the specific ions after normalization and calibration with the appropriate internal and external standards [epicoprostanol m/z 370, 2H7-7-lathosterol m/z 465, 2H6-desmosterol m/z 358, 2H6-lanosterol m/z 504, 2H7-24(R/S)-hydroxycholesterol m/z 553, cholesterol m/z 329, 7-lathosterol m/z, 7-dehydrocholesterol m/z 325, 8-dehydrocholesterol m/z 325, desmosterol m/z 343, lanosterol m/z 393 and 24(R/S)-hydroxycholesterol m/z 413].

### Immunofluorescence quantitative analysis of neuronal survival

Neuronal survival was evaluated through in direct quantification of cells HA positive in the diverse conditions in 5 wells par condition at magnification 10x on the Leica microscope.

### Statistical analysis

Statistical analysis was performed using unpaired Student's t-test. Results are expressed as mean ± SEM. Significant thresholds were set at P<0.05, P<0.01 and P<0.001, as defined in the text. All analyses were performed using GraphPad Prism (GraphPad Software, La Jolla, USA).

### Results

### Basal evaluation of cholesterol pathway in SOD1 animals

Levels of expression of several gene of the cholesterol pathway have been quantified in lumbar spinal cord of 8 weeks of SOD1^{G93A} and WT animals (Figure 1). A significant decrease of CYP46A1 and ApoE have been observed (Figure 1A) and a trend for SREBP1, SREBP2 and HMGCR (Figure 1A). In addition, measurement of 24-hydroxycholesterol has been done at 15 weeks of age in lumbar spinal cord of SOD1^{G93A} and WT animals demonstrating an important decrease (around 60% diminution) of its content in SOD1^{G93A} compared to WT animals (Figure 1B). These results confirm the hypothesis of modulating CYP46A1 in ALS models too rescue ALS phenotype.

### Validation of AAVPHP.eB as a good vector for ALS

The inventors investigate first whether AAVPHP.eB is a good vector for ALS. For this purpose; 8 weeks-old WT animals have been injected with low (2.5.1011vg), medium (5.1011vg) or high (1.1012vg) doses of AAVPHP.eB encoding CYP46A1-HA. A good targeting of motoneurons has been highlighted with the 3 doses (Figure 2) without major differences between low and high doses and a transduction rate of 50 to 60% (Figure 2).

This large transduction is not associated with any immune response in the spinal cord even in the high dosed group as assessed with GFAP (Figure 3) and Iba1 (non-presented data) staining evaluation that did not lead to any microgliosis or astrogliosis (Figure 3).

These results motivated to pursue with the low dose of vector.

### Prevention of ALS phenotype in SOD1G93A mouse model of ALS

CYP46A1 overexpression prevent behavioral alterations in a mouse model of ALS with preventive treatment.

The inventors investigated whether the upregulation of the cholesterol metabolism pathway, through increase in the levels of CYP46A1, could improve motor alteration in an in vivo model of ALS: the SOD1G93A model. Overexpression of CYP46A1 by preventive intravenous administration of AAVPHP.eB-CYP46A1-HA in 3 weeks old mice clearly prevent motor alteration in the mouse model. First, the AAV treated SOD1G93A mice have an improved growth curve (Figure 4A). Preventive AAVPHP.eB-CYP46A1-HA delivery significantly corrects motor impairment measured by clasping test (Figure 4B) and fully prevents alteration measured by inverted test (Figure 4C).

Biodistribution study revealed around 1 vector genome copy (VGC) in all levels of the spinal cord and between 2 and 4 copies in the brain (Figure 5A) as well as a really low transduction of the peripheral tissues with a maximum of 0.4 VGC in the heart and less that 0.1 VGC in the other peripheral organs (Figure 5B). Target engagement has been validated with the quantification of 24 hydroxycholesterol in the lumbar spinal cord of the animals, revealing a 3-fold increase in treated animals compared to WT animals (Figure 5C).

This increase in 24 OH cholesterol perfectly correlates with a large expression of CYP46A1-HA in all levels of the spinal cord, especially in motoneurons (MNs) (Figure 6A), that also confirmed the results previously obtained in WT animals (Figure 2).

A partial preservation of the MNs has been demonstrated at the lumbar level (around 60% compared to WT), assessed by quantification of co-staining with VachT and HA (Figure 6B and C), similar number of MNs in the thoracic section compared to WT and no difference at the cervical level (Figure 6C). This 60% preservation is sufficient to avoid demyelination of the lumbar spinal cord, as demonstrated with Luxol staining and quantification of myelin percentage (Figure 6D and E). AAV-treated SOD1 animals had a myelin percentage equal to WT animals and significantly higher than SOD1 non treated animals.

The other aspect inventors wanted to investigate was the muscle phenotype, indeed, muscles are heavily affected in ALS, mainly due to the loss of innervation and neuromuscular junctions (NMJs), consecutive to the loss of MNs.

They first demonstrated a significant preservation of muscle structure assessed by measurement of mean fiber area (Figure 7A). Tibialis muscle of SOD1-treated animal display a significant increase of mean fiber area compared to non-treated animals (Figure 7B) and repartition according fiber cross sectional area, clearly demonstrated the preservation of large cross section fibers (Figure 7B). Similar results have been observed for gastrocnemius (Figure 7D and E) and quadriceps (Figure 7F and 7G).

Then, they focus on NMJs phenotype, demonstrating an increase number of innervated junctions in treated animals compared to non-treated SOD1 animals (Figure 8A and B), as well as an improved structure of the NMJs with notably a higher number of NMJs with normal endplate or thicker endplate and a decreased number of ectopic and fragmented endplates (Figure 8C).

This result is particularly important and with therapeutic significant, because when inventors evaluated the state of NMJs at 3 weeks in WT and SOD1G93A non treated animals, NMJs revealed to be already pathologic at 3 weeks with an increase number of immature junctions in SOD1 animals compared to WT (Figure 8D and E). This means that even when the AAV-CYP46A1 is injected when NMJs are not properly formed, it is still able to significantly improve the phenotype of the treated animals.

To complete the study, the inventors also quantified levels of expression of Musk, which is the receptor involved in the binding with agrin and nAchR, the receptor of Acetylcholine, both involved in the stabilization of synapses and the maintenance of NMJs, in Tibialis muscle at 15 weeks of age. Both MuSK and nAchR levels of expression are improved in tibialis and gastrocnemius muscles (Figure 9).

### CYP46A1 overexpression alleviates behavioral alterations in a mouse model of ALS after curative treatment

Based on preventive treatment in animals, investigations were performed to determine whether the upregulation of the cholesterol metabolism pathway, through increase in the levels of CYP46A1, could improve motor alteration in a post symptomatic in vivo model of ALS: the SOD1G93A model. Overexpression of CYP46A1 by intravenous administration of AAVPHP.eB-CYP46A1-HA in 8 weeks old mice clearly alleviate motor alteration in the mouse model. Mice have an improved growth compared to non-treated animals (Figure 10A).

Curative AAVPHP.eB-CYP46A1 administration clearly decreases motor impairment measure by clasping test (Figure10B) and alleviate alteration measured by inverted test (Figure 10C). In addition, the treatment, lead to an increased survival of the treated mice compared to non-treated animals with an average of 14 days life expectancy increase (Figure 10D).

Mice were euthanized at 15 weeks of age for histological and molecular analysis.

Biodistribution study, revealed around 3-4 vector genome copy (VGC) in all levels of the spinal cord and between 10 and 20 copies in the brain (Figure 11A) as well as a really low transduction of the peripheral tissues with a maximum of 0.5 VGC in the liver and less that 0.2 VGC in the other peripheral organs (Figure 11B). Target engagement has been validated with the quantification of 24 hydroxycholesterol in the lumbar spinal cord of the animals, revealing a 1.3-fold increase in treated animals compared to WT animals (Figure 11C).

A partial preservation of the MNs has been demonstrated at the lumbar level (around 60% compared to WT), assessed by quantification of co-staining with VachT and HA (Figure 12A and B), similar results to the preventive treatment (Figure 6C).

This 60% preservation is here again sufficient to avoid demyelination of the lumbar spinal cord, as demonstrated with Luxol staining and quantification of myelin percentage (Figure 12C). AAV-treated SOD1 animals had a myelin percentage equal to WT animals and significantly higher than SOD1 non treated animals.

Muscle structure assessment by measurement of mean fiber area (Figure 13) revealed a clear improvement of the phenotype in treated animals compared to non-treated animals. Quadriceps muscle of SOD1-treated animal display a significant increase of mean fiber area compared to non-treated animals (Figure 13E and F). Similar results have been observed for tibialis (Figure 13A and B) gastrocnemius (Figure 13 C and D).

Then, they focus on NMJs phenotype, demonstrating an increase number of innervated junction in treated animals compared to non-treated SOD1 animals (Figure 14A and B), as well as an improve structure of the NMJs with notably an higher number of NMJs with normal endplate and a decrease number of fragmented and non-mature endplates (Figure 14C).

Finally, effect of treatment on both MuSK and nAchR levels of expression are milder than in preventive treatment (Figure 15) but improved as in gastrocnemius muscles (Figure 15D).

Altogether, these data strongly support CYP46A1 as a relevant target in ALS. The inventors then decided to evaluate if increasing AAV-CYP46A1 dose could still improve the beneficial effect and a cohort of SOD1G93A animals has been injected at 5.1011vg with curative treatment. Preliminary results on behavior suggest a strong effect and a clear improvement of the behavior measured with clasping score (Figure 16). Mice are at 12 weeks and will be evaluated for survival.

Finally, the inventors claimed that CYP46A1 could be a relevant target for both familial and sporadic forms of ALS, thus targeting not only SOD1 mutated patients but also C9ORF72 mutated patients of TARDP patients. For that purpose, inventors decided to test their therapeutic strategy on other models of ALS, the first one is the C9ORF72 mouse model with 500 repetitions of GGGGCC.

The inventors administered 5.1011vg AAV-CYP46A1-HA as a preventive treatment at 4 weeks in C9ORF72 models and currently a follow up until 16 weeks has been done. No difference in term of growth curve has been observed (Figure 17A). A clear improvement of the motor performances has been demonstrated based on the notched bar test (Figure 17B). Mice follow up is ongoing and survival will be assessed.

Overall, these data support that CYP46A1 overexpression has therapeutic properties, promoting improvement of neuronal physiology, especially autophagy in an in vitro model of ALS (Figure 18) and data on post symptomatic delivery of the treatment is encouraging for treatment of ALS when symptoms are already developed.

### References

1. Zarei S, Carr K, Reiley L, et al. A comprehensive review of amyotrophic lateral sclerosis. Surg Neurol Int 2015;6:171.
2. Rowland LP, Shneider NA. Amyotrophic Lateral Sclerosis. N Engl J Med 2001;344:1688-1700.
3. Greenway MJ, Andersen PM, Russ C, et al. ANG mutations segregate with familial and "sporadic" amyotrophic lateral sclerosis. Nat Genet 2006;38:411-413.
4. Kabashi E, Valdmanis PN, Dion P, et al. TARDBP mutations in individuals with sporadic and familial amyotrophic lateral sclerosis. Nat Genet 2008;40:572-574.
5. Kaur SJ, McKeown SR, Rashid S. Mutant SOD1 mediated pathogenesis of Amyotrophic Lateral Sclerosis. Gene 2016;577:109-118.
6. Turner MR, Al-Chalabi A, Chio A, et al. Genetic screening in sporadic ALS and FTD. J Neurol Neurosurg Psychiatry 2017;88:1042-1044.
7. Ticozzi N, Vance C, LeClerc AL, et al. Mutational analysis reveals the FUS homolog TAF15 as a candidate gene for familial amyotrophic lateral sclerosis. Am J Med Genet Part B Neuropsychiatr Genet 2011;156:285-290.
8. Valdmanis PN, Rouleau GA. Genetics of familial amyotrophic lateral sclerosis. Neurology 2008;70:144-152.
9. Zoccolella S, Beghi E, Palagano G, et al. Riluzole and amyotrophic lateral sclerosis survival: a population-based study in southern Italy. Eur J Neurol 2007;14:262-268.
10. Brooks BR, Jorgenson JA, Newhouse BJ, et al. Edaravone in the treatment of amyotrophic lateral sclerosis: efficacy and access to therapy - a roundtable discussion. Am J Manag Care 2018;24:S175-S186.
11. Prudencio M, Belzil V V, Batra R, et al. Distinct brain transcriptome profiles in C9orf72-associated and sporadic ALS. Nat Neurosci 2015;18:1175-82.
12. Björkhem I, Lütjohann D, Diczfalusy U, et al. Cholesterol homeostasis in human brain: turnover of 24S-hydroxycholesterol and evidence for a cerebral origin of most of this oxysterol in the circulation. J Lipid Res 1998;39:1594-600.
13. Chan KY, Jang MJ, Yoo BB, et al. Engineered AAVs for efficient noninvasive gene delivery to the central and peripheral nervous systems. Nat Neurosci 2017;20:1172-1179.
14. Mast N, Zheng W, Stout CD, et al. Antifungal Azoles: Structural Insights into Undesired Tight Binding to Cholesterol-Metabolizing CYP46A1. Mol Pharmacol 2013;84:86-94.
15. Mast N, Anderson KW, Johnson KM, et al. In vitro cytochrome P450 46A1 (CYP46A1) activation by neuroactive compounds. J Biol Chem 2017;292:12934-12946.
16. Mast N, Li Y, Linger M, et al. Pharmacologic Stimulation of Cytochrome P450 46A1 and Cerebral Cholesterol Turnover in Mice. J Biol Chem 2014;289:3529-3538.
17. Mast N, Saadane A, Valencia-Olvera A, et al. Cholesterol-metabolizing enzyme cytochrome P450 46A1 as a pharmacologic target for Alzheimer's disease. Neuropharmacology 2017;123:465-476.
18. Fourgeux C, Martine L, Acar N, et al. In vivo consequences of cholesterol-24S-hydroxylase (CYP46A1) inhibition by voriconazole on cholesterol homeostasis and function in the rat retina. Biochem Biophys Res Commun 2014;446:775-781.
19. Sellier C, Campanari M-L, Julie Corbier C, et al. Loss of C9ORF72 impairs autophagy and synergizes with polyQ Ataxin-2 to induce motor neuron dysfunction and cell death. EMBO J 2016;35:1276-97.
20. Hudry E, Van Dam D, Kulik W, et al. Adeno-associated virus gene therapy with cholesterol 24-hydroxylase reduces the amyloid pathology before or after the onset of amyloid plaques in mouse models of Alzheimer's disease. Mol Ther 2010;18:44-53.
21. Piguet F, de Montigny C, Vaucamps N, et al. Rapid and Complete Reversal of Sensory Ataxia by Gene Therapy in a Novel Model of Friedreich Ataxia. Mol Ther . Epub ahead of print May 28, 2018. DOI: 10.1016/j.ymthe.2018.05.006.
22. Charvin D, Vanhoutte P, Pages C, et al. Unraveling a role for dopamine in Huntington's disease: The dual role of reactive oxygen species and D2 receptor stimulation. Proc Natl Acad Sci 2005;102:12218-12223.
23. Deyts C, Galan-Rodriguez B, Martin E, et al. Dopamine D2 Receptor Stimulation Potentiates PolyQ-Huntingtin-Induced Mouse Striatal Neuron Dysfunctions via Rho/ROCK-II Activation. PLoS One 2009;4:e8287.
24. Audouard E, Schakman O, René F, et al. The Onecut Transcription Factor HNF-6 Regulates in Motor Neurons the Formation of the Neuromuscular Junctions. PLoS One 2012;7:e50509.
25. Dzeletovic S, Breuer O, Lund E, et al. Determination of Cholesterol Oxidation Products in Human Plasma by Isotope Dilution-Mass Spectrometry. Anal Biochem 1995;225:73-80.
26. Chevy F, Humbert L, Wolf C. Sterol profiling of amniotic fluid: a routine method for the detection of distal cholesterol synthesis deficit. Prenat Diagn 2005;25:1000-1006.

## Claims

1. A vector for use in the treatment of amyotrophic lateral sclerosis (ALS), which vector comprises cholesterol 24-hydroxylase encoding nucleic acid.

2. The vector for use in the treatment of ALS according to claim 1, wherein the ALS is associated with at least one motor neurons related disorder.

3. The vector for use in the treatment of ALS according to claim 2, wherein the ALS is associated with frontotemporal dementia.

4. The vector for use in the treatment of ALS according to any one of claims 1 to 3, comprising a nucleic acid sequence that encodes the amino acid sequence SEQ ID N°2.

5. The vector for use in the treatment of ALS according to any one of claims 1 to 4, comprising the nucleic acid sequence SEQ ID N°1.

6. The vector for use in the treatment of ALS according to any one of claims 1 to 5, which is selected from the group of adenovirus, lentivirus, retrovirus, herpesvirus and Adeno-Associated Virus (AAV) vectors.

7. The vector for use in the treatment of ALS according to any one of claims 1 to 6, which is an AAV vector.

8. The vector for use in the treatment of ALS of claim 7, which is an AAV9, AAV10 vector, such as AAVrh.10, or AAVPHP.eB, preferably an AAVPHP.eB.

9. The vector for use in the treatment of ALS according to any one of claims 1 to 8, which is to be administered directly intravenous or into the brain of the patient.

10. The vector for use in the treatment of ALS of claim 9, which is to be administered to spinal cord and/or motor cortex.

11. The vector for use in the treatment of ALS of claim 10, which is to be administered to motoneurons.

12. The vector for use in the treatment of ALS according to any one of claims 1 to 11, which is to be administered by intravascular, intravenous, intranasal, intraventricular or intrathecal injection.

13. A pharmaceutical composition for use in the treatment of amyotrophic lateral sclerosis, which comprises a therapeutically effective amount of a vector as defined in any one of claims 1 to 12.

## Patentansprüche

1. Vektor zur Verwendung bei der Behandlung von amyotropher Lateralsklerose (ALS), wobei der Vektor für Cholesterin-24-Hydroxylase kodierende Nukleinsäure umfasst.

2. Vektor zur Verwendung bei der Behandlung von ALS nach Anspruch 1, wobei die ALS mit mindestens einer mit motorischen Neuronen zusammenhängenden Störung assoziiert ist.

3. Vektor zur Verwendung bei der Behandlung von ALS nach Anspruch 2, wobei die ALS mit frontotemporaler Demenz assoziiert ist.

4. Vektor zur Verwendung bei der Behandlung von ALS nach einem der Ansprüche 1 bis 3, umfassend eine Nukleinsäuresequenz, die die Aminosäuresequenz SEQ ID Nr. 2 kodiert.

5. Vektor zur Verwendung bei der Behandlung von ALS nach einem der Ansprüche 1 bis 4, umfassend die Nukleinsäuresequenz SEQ ID Nr. 1.

6. Vektor zur Verwendung bei der Behandlung von ALS nach einem der Ansprüche 1 bis 5, der ausgewählt ist aus der Gruppe der Adenovirus-, Lentivirus-, Retrovirus-, Herpesvirus- und Adeno-assoziierten Virus (AAV)-Vektoren.

7. Vektor zur Verwendung bei der Behandlung von ALS nach einem der Ansprüche 1 bis 6, der ein AAV-Vektor ist.

8. Vektor zur Verwendung bei der Behandlung von ALS nach Anspruch 7, der ein AAV9-, AAV10-Vektor, wie AAVrh.10, oder AAVPHP.eB, vorzugsweise ein AAVPHP.eB, ist.

9. Vektor zur Verwendung bei der Behandlung von ALS nach einem der Ansprüche 1 bis 8, der direkt intravenös oder in das Gehirn des Patienten zu verabreichen ist.

10. Vektor zur Verwendung bei der Behandlung von ALS nach Anspruch 9, der an das Rückenmark und/oder den motorischen Cortex zu verabreichen ist.

11. Vektor zur Verwendung bei der Behandlung von ALS nach Anspruch 10, der Motoneuronen zu verabreichen ist.

12. Vektor zur Verwendung bei der Behandlung von ALS nach einem der Ansprüche 1 bis 11, der durch intravaskuläre, intravenöse, intranasale, intraventrikuläre oder intrathekale Injektion zu verabreichen ist.

13. Pharmazeutische Zusammensetzung zur Verwendung bei der Behandlung von amyotropher Lateralsklerose, die eine therapeutisch wirksame Menge eines Vektors, wie in einem der Ansprüche 1 bis 12 definiert, umfasst.

## Revendications

1. Vecteur pour son utilisation dans le traitement de la sclérose latérale amyotrophique (SLA), lequel vecteur comprend un acide nucléique codant pour la cholestérol 24-hydroxylase.

2. Vecteur pour son utilisation dans le traitement de la SLA selon la revendication 1, dans lequel la SLA est associée à au moins une maladie liée aux neurones moteurs.

3. Vecteur pour son utilisation dans le traitement de la SLA selon la revendication 2, dans lequel la SLA est associée à la démence fronto-temporale.

4. Vecteur pour son utilisation dans le traitement de la SLA selon l'une quelconque des revendications 1 à 3, comprenant une séquence d'acide nucléique qui code pour la séquence d'amino-acide SEQ ID N°2.

5. Vecteur pour son utilisation dans le traitement de la SLA selon l'une quelconque des revendications 1 à 4, comprenant la séquence d'acide nucléique SEQ ID N°1.

6. Vecteur pour son utilisation dans le traitement de la SLA selon l'une quelconque des revendications 1 à 5, qui est sélectionné parmi le groupe de vecteurs des adénovirus, lentivirus, rétrovirus, herpèsvirus et les virus adénoassociés (AAV).

7. Vecteur pour son utilisation dans le traitement de la SLA selon l'une quelconque des revendications 1 à 6, qui est un vecteur AAV.

8. Vecteur pour son utilisation dans le traitement de la SLA selon la revendication 7, qui est un vecteur AAV9, AAV10 par exemple AAVrh.10, ou AAVPHP.eB, préférentiellement AAVPHP.eB.

9. Vecteur pour son utilisation dans le traitement de la SLA selon l'une quelconque des revendications 1 à 8, qui est destiné à être administré par voie intraveineuse ou dans le cerveau du patient.

10. Vecteur pour son utilisation dans le traitement de la SLA selon la revendication 9, qui est destiné à être administré à la moelle épinière et/ou au cortex moteur.

11. Vecteur pour son utilisation dans le traitement de la SLA selon la revendication 10, qui est destiné à être administré dans les motoneurones.

12. Vecteur pour son utilisation dans le traitement de la SLA selon l'une quelconque des revendications 1 à 11, qui est destiné à être administré par injection intravasculaire, intraveineuse, intranasale, intraventriculaire ou intrathécale.

13. Une composition pharmaceutique pour son utilisation dans le traitement de la sclérose latérale amyotrophique, qui comprend une quantité thérapeutiquement efficace d'un vecteur tel que défini dans l'une quelconque des revendications 1 à 12.
